# EUROPÄISCHE PATENTSCHRIFT

(11) **EP 1 886 640 B1**
(45) Veröffentlichungstag und Bekanntmachung des Hinweises auf die Patenterteilung: **18.11.2009**
(21) Anmeldenummer: 06016532.1
(22) Anmeldetag: 08.08.2006
(51) Int. Cl.: A61B 19/00

(54) **Planungsverfahren und System zur Freiform-Implantatsanpassung**
Planning method and system for adjusting a free-shaped bone implant
Système et méthode de planification pour l'ajustement d'un implant osseux de forme libre

(43) Veröffentlichungstag der Anmeldung: 13.02.2008
(73) Patentinhaber: BrainLAB AG, 85622 Feldkirchen (DE)
(72) Erfinder: Neubauer, Timo, 85586 Poing (DE); Millahn, Manuel, 80799 München (DE)
(74) Vertreter: Rögner, Jürgen

(56) Entgegenhaltungen:
- EP-A2- 0 456 103
- DE-A1- 19 904 640
- US-A- 5 879 354
- US-A1- 2003 233 094
- US-A1- 2004 243 134
- US-A1- 2005 267 584
- US-A1- 2006 058 883

## Beschreibung

Die Erfindung betrifft ein Planungsverfahren und ein System zur Anpassung eines Knochenimplantates.

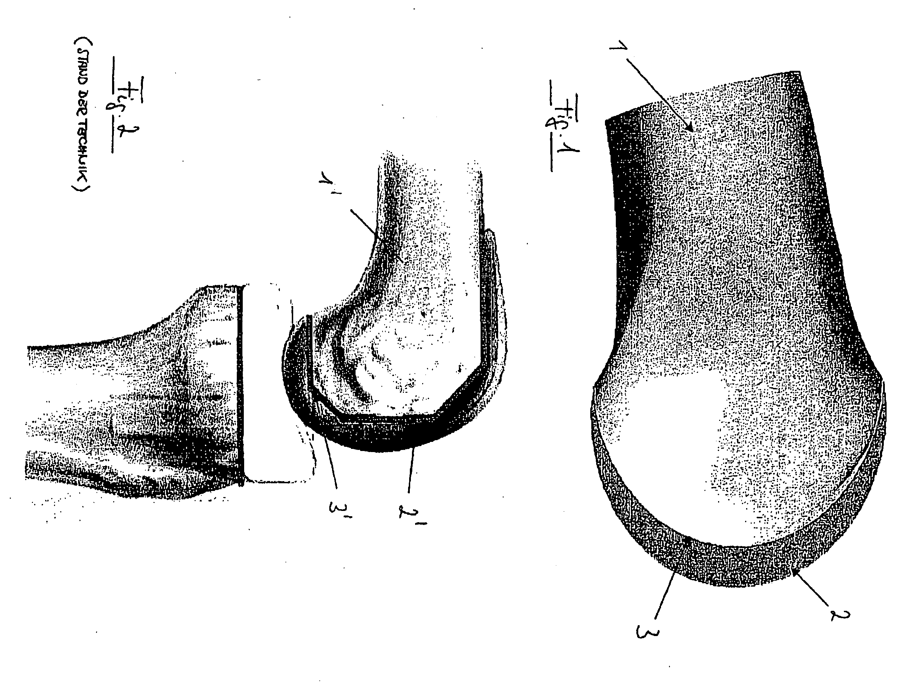

Um einen Gelenkersatz im menschlichen Körper bereitstellen zu können, ist es notwendig, den Knochen so vorzubereiten, dass er zum Implantat passt. Das Implantat muss die Anforderungen an die Bio-Funktionalität erfüllen und deshalb genau angeordnet und ausgerichtet werden. In den Zeiten, in denen noch keine bildunterstützte bzw. navigationsunterstützte Chirurgie zur Verfügung stand, bestand die einzige Möglichkeit der Ausrichtung eines Implantates darin, seine Anpassung durch das Erzeugen von fünf Ebenen vorzudefinieren, und zwar mit Hilfe unterschiedlicher Positionierungsinstrumente wie zum Beispiel eines Marknagels. Diese Technik ist in der beiliegenden Figur 2 veranschaulicht, die einen Knochen 1' zeigt, der an einem Ebenenzug abgetragen wurde, der fünf Ebenen aufweist und das Bezugszeichen 3' zeigt. Ein mit einer entsprechenden Innenkontur versehenes Implantat 2' wurde dann auf den Knochen aufgesetzt. Einerseits war diese Methode sehr invasiv, das heißt es wurde sehr viel Knochenmaterial abgetragen; andererseits war die Ausrichtung des Implantats oftmals nicht sehr genau.

Heutzutage werden navigierte Schneidblöcke verwendet, mit Hilfe derer wiederum vordefinierte Flächen am Knochen abgeschnitten bzw. erzeugt werden. Damit wird die Ausrichtung des Implantates verbessert, aber durch die glatten Schnitte wird noch immer viel Knochenmaterial weggenommen.

Aus der EP 0 456 103 A2 ist ein Verfahren zum Abtrennen von Knochengewebe bekannt, so dass anschließend ein Implantat gesetzt werden kann. Dabei kann anhand bildgebender Verfahren ein passendes Implantat ausgewählt werden.

Die US 2003/0,233,094 A1 zeigt eine Vorrichtung zum Abnehmen von Knochenmaterial, so dass eine Kontaktstelle zum Anbringen von einem Steg oder einer Platte zum Fixieren von Wirbelkörpern zueinander entsteht.

Die DE 199 04 640 A1 zeigt ein Verfahren zum Trennen oder Entfernen eines Knochens mit einer Wasserstrahlschneidanlage.

Es ist die Aufgabe der vorliegenden Erfindung, ein Planungsverfahren bzw. ein System zur Anpassung eines Knochenimplantates zu schaffen, mit denen die oben angesprochenen Nachteile des Standes der Technik überwunden werden. Insbesondere soll eine Planung aufgezeigt werden, die es gestattet, möglichst wenig vom gesunden bzw. noch brauchbaren Knochenmaterial abzunehmen und trotzdem ein festsitzendes, gut angepasstes und gut ausgerichtetes Implantat zu setzen.

Diese Aufgabe wird durch ein Planungsverfahren gemäß dem Anspruch 1 sowie durch ein System gemäß dem Anspruch 7 gelöst. Die Unteransprüche definieren bevorzugte Ausführungsformen der Erfindung.

Die Erfindung stellt ein Planungsverfahren bereit, das zur Anpassung eines Knochenimplantates dient und bei dem
- der Zustand eines zu behandelnden Knochens durch ein Röntgen-, Computertomographie- oder Magnetresonanztomographieverfahren, ermittelt wird;
- durch eine computergraphisch gestützte Segmentierung ein disfunktionaler Teil des Knochens bestimmt wird;
- diejenige Fläche des Knochens als Fläche mit freier Form ermittelt und registriert wird, die gerade unterhalb der Grenzfläche zwischen gesunden Knochengewebe und dem disfunktionalen Teil liegt; und bei dem
- ein Steuerungsdatensatz erstellt wird, welcher der Abtragung des Knochens an der ermittelten Freiformfläche durch eine navigationstechnisch unterstützte Abtragungsvorrichtung zugrunde gelegt werden kann.

Zur Ermittlung des Knochenzustandes sind auch andere bildgebende Verfahren als die genannten denkbar. Der Unterschied zum Stand der Technik liegt also insbesondere darin, dass das Planungsverfahren die Herstellung einer freien Formfläche unterstützt, und eine solche freie Formfläche kann sehr viel flexibler gestaltet werden als vorgeschriebene und flache bzw. gerade Ebenen, wie sie mit Schneidblöcken erzielbar sind. Durch diese freiere Gestaltungsmöglichkeit kann sehr viel Knochenmaterial, das noch funktional ist, geschont werden. Bei Revisions-Operationen steht dieses Knochenmaterial dann wieder zur Verfügung, um zusätzlichen Halt zu geben.

Die ermittelte und registrierte Freiformfläche kann in unterschiedlicher Weise und zweckgebunden ausgebildet sein. So ist es einerseits möglich, die Fläche in einer solchen Weise zu ermitteln und zu registrieren, dass sie gerade unterhalb der Grenzefläche zwischen gesundem Knochengewebe und disfunktionalem Teil liegt. Damit würde am meisten Knochen geschont und die Implantat-Kontaktseite könnte entsprechend an die neue Freiform-Oberfläche angepasst werden.

Es ist möglich, eine der Freiformfläche am Knochen entsprechende Gegenfläche am Implantat auszubilden, und damit bietet die vorliegende Erfindung den Vorteil einer automatischen Ausrichtung des Implantates, weil auch die schon im Planungsverfahren berücksichtigte Freiformfläche automatisch als Gegenstück im Implantat nachgeformt werden kann. Solche Freiformflächen ermöglichen einen besseren Halt des Implantates, eine bessere Lastverteilung aufgrund der besseren Anpassung, und sie bringen, wie schon erwähnt, weniger Komplikationen bei Revisionsbehandlungen mit sich. Außerdem können sie auch vorgeformte Implantat-Innenseiten unterstützen bzw. an diese angepasst werden.

Bei einer Ausführungsform des erfindungsgemäßen Verfahrens wird der Steuerungsdatensatz für die Führung einer mit freier Hand geführten Abtragungsvorrichtung erstellt, die mittels eines medizintechnischen Tracking-Systems positionell erfasst und verfolgt wird. Dabei werden auf der Basis des Steuerungsdatensatzes unterstützende Informationen und Anweisungen für die Führung der Abtragungsvorrichtung, die insbesondere über eine Bildausgabe ausgebbar sind, durch ein dem Tracking-System zugeordnetes medizintechnisches Navigationssystem erstellt.

Gemäß einer weiteren Ausführung des Verfahrens gemäß der Erfindung wird der Steuerungsdatensatz für die Führung einer von Hand führbaren und mechanisch gehalterten Abtragungsvorrichtung erstellt, die mittels eines medizintechnischen Trackings-Systems positionell erfasst und verfolgt wird. Hierbei werden auf der Basis des Steuerungsdatensatzes unterstützende Informationen, Anweisungen sowie insbesondere auch mechanische Führungshilfen für die Führung der Abtragungsvorrichtung durch ein dem Tracking-System zugeordnetes medizintechnisches Navigationssystem erstellt.

Bei einer weiteren Ausführungsvariante wird der Steuerungsdatensatz für die Führung einer mechanisch gehalterten und geführten Abtragungsvorrichtung erstellt, die mittels eines medizintechnischen Tracking-Systems positionell erfasst und verfolgt wird, wobei auf der Basis des Steuerungsdatensatzes Steuerungsanweisungen für die Führung der Abtragungsvorrichtung durch ein dem Tracking-System zugeordnetes medizintechnisches Navigationssystem erstellt werden.

Bei den beiden oben zuletzt genannten Fällen besteht die Möglichkeit, den Steuerungsdatensatz für die mechanische Halterung bzw. Führung für einen chirurgischen Roboter zu erstellen, der die Abtragungsvorrichtung trägt. Ferner kann der Steuerungsdatensatz für eine Abtragungsvorrichtung erstellt werden, die ein Schneidwerkzeug, ein Strahlschneidwerkzeug, ein Bohrwerkzeug, ein Fräswerkzeug, ein Laser-Abtragungswerkzeug oder eine Kombination aus zweien oder mehreren dieser Werkzeuge aufweist.

Ein erfindungsgemäßes System zur Anpassung eines Knochenimplantates ist ein System mit

Mitteln zur Ermittlung des Zustandes eines zu behandelnden Knochens (1) durch ein bildgebendes Verfahren;
- einer Datenverarbeitungseinrichtung welcher die Abtragung eines Knochens an einher Freiformfläche (3) steuert, mit Mitteln zur Erstellung eines Steuerungsdatensatzes,
- Mitteln zur Bestimmung eines disfunktionalen Knochenteils durch computergraphisch gestützte Segmentierung, und mit
- einer navigationstechnisch unterstützten Knochen-Abtragungsvorrichtung (4), welche den Knochen (1) an der Freiformfläche (3) abtragen kann, **dadurch gekennzeichnet dass** die Datenverarbeitungseinrichtung Mitteln zur Ermittlung und Registrierung der Freiformfläche, aufweist, die gerade unterhalb der Grenzfläche zwischen gesundem Knochengewebe und dem durch eine computergraphisch gestützte Segmentierung bestimmten disfunktionalen Knochenteil liegt.

Die schon oben für das Planungungsverfahren genannten Vorteile treffen natürlich auch für das erfindungsgemäße System zu.

Bei einer Ausführungsform der Erfindung umfasst das System ein medizintechnisches Tracking-System, mit dem die Abtragungsvorrichtung positionell erfasst und verfolgt wird, sowie ein dem Tracking-System zugeordnetes medizintechnisches Navigationssystem, das auf der Basis des Steuerungsdatensatzes eine Führungsunterstützung für die Abtragungsvorrichtung bereitstellt. Die Abtragungsvorrichtung kann eine mit freier Hand geführte Abtragungsvorrichtung, eine von Hand führbare und mechanisch gehalterte Abtragungsvorrichtung oder eine mechanisch gehalterte und geführte Abtragungsvorrichtung sein. Sie kann von einem chirurgischen Roboter getragen werden, der sie mechanisch haltert bzw. führt, und sie kann ein Schneidwerkzeug, ein Bohrwerkzeug, ein Fräswerkzeug, ein Laser-Abtragungswerkzeug, ein Strahlschneidewerkzeug oder eine Kombination aus zweien oder mehreren dieser Werkzeuge aufweisen. Natürlich sind diese Werkzeuge hier nur beispielsweise genannt, alle Werkzeuge, die sich dazu eignen, Substanz an einem Knochen abzutragen, sind für die Verwendung mit der vorliegenden Erfindung geeignet. Das System kann eine Implantat-Bearbeitungsvorrichtung umfassen, die einen Knochen-Anlageabschnitt eines Knochenimplantats als passendes Gegenstück zur Freiformfläche ausbilden kann.

Da bei einem Planungsverfahren und einem System gemäß der vorliegenden Erfindung die Ausrichtung des Implantates nicht durch Ebenen vordefiniert werden muss sondern durch die Führung des Navigationssystems realisiert werden kann, kann also mit anderen Worten geplant werden, einen "Offset" bzw. einen Versatz gegenüber der disfunktionalen oder zerstörten Knochenoberfläche zu erzeugen, das heißt die disfunktionale Oberfläche bzw. den disfunktionalen Knochenbereich abzutragen und eine neue Oberfläche darunter zu generieren, die aus tragendem Knochenmaterial besteht. Die Innenseite des Implantates kann eventuell entsprechend angepasst werden.

Die Erfindung verbessert also die Planung der Vorbereitung sowie die Vorbereitung des Knochens bei dessen Anpassung an endoprothetische Implantate. Die bisher verwendete Knochensäge, die flache ebenenartige Abschnitte herstellte, wird durch eine andere Technologie ersetzt, wie zum Beispiel Laserschneiden, Strahlschneiden, Hybrid-Laserschneiden oder Fräsen.

Wenn schließlich aufgrund der erfindungsgemäßen Planung oder mit Hilfe des erfindungsgemäßen Systems die Operation durchgeführt wird, kann der Chirurg alle für den Implantationsvorgang relevanten Knochen registrieren (im Navigationssystem positionell zuordnen und identifizieren), und dies beispielsweise bei einer Gesamt-Knieersatz-Operation, oder bei Operationen am Ober- oder Unterschenkel. Hierzu können verschiedene Techniken eingesetzt werden, beispielsweise eine CT-freie Registrierung mit einem navigierten Pointerinstrument oder eine CT/MR-basierte Registrierung, die CT/MR-Bilder verwendet. Andere Registrierungstechniken sind ebenfalls einsetzbar. Um die im Vorfeld vom Planungssystem generierte Oberfläche zu erzeugen, die gerade soweit unterhalb der realen Oberfläche liegt, dss das gesamte disfunktionale Gewebe entfernt wird, kann das Abtragungswerkzeug an einem aktiven oder passiven Roboterarm angesetzt werden, der - unterstützt durch die Planung - eine Führung für das Werkzeug bereitstellt oder das Werkzeug selbst führt. Wenn die Biofunktionalität beeinträchtigt ist, wird die Oberfläche durch das Planungssystem schon so verschoben, dass die Ausrichtung des Implantats wieder eine korrekte Biofunktionalität unterstüzt.

Die Erfindung wird nun anhand der beiliegenden Zeichnungen und unter Bezugnahme auf Ausführungsformen näher erläutert. Sie kann alle hierin beschriebenen Merkmale einzeln oder in jedweder sinnvollen Kombination umfassen. In den Zeichnungen zeigen:
- Figur 1: einen Knochen mit einem Implantat und einer erfindungsgemäßen Freiform-Anpassung;
- Figur 2: eine Implantatanpassung gemäß dem Stand der Technik;
- Figuren 3 und 4: verschiedene Implantatformen mit Freiform-Anpassungsflächen; und
- Figur 5: eine Darstellung eines erfindungsgemäßen Systems zur Freiform-Implantatanpassung.

Die Figur 1 zeigt schon einen Knochen, an den erfindungsgemäß ein Implantat angepasst worden ist. Der Knochen trägt das Bezugszeichen 1, das Implantat das Bezugszeichen 2. Das Implantat 2 ist außen rund ausgestaltet und hat an seiner Innenseite eine Form, die genau an die Freiformfläche 3 angepasst ist, die an dem Knochen durch ein erfindungsgemäßes System ausgebildet worden ist, wobei das erfindungsgemäße Planungsverfahren zugrunde gelegt wurde. Man erkennt, dass die Knochenoberfläche im Bereich des Implantats nicht mehr aus einem Zug glatter Ebenen besteht, wie dies gemäß dem Stand der Technik der Fall war (siehe Figur 2), sondern eine freie Form aufweist, die an jeder Stelle an der Oberfläche 3 tragfähiges, gesundes und funktionales Knochenmaterial zeigt. Man erkennt beim Vergleich der Figuren 1 und 2 durchaus auch, wie sehr das Knochenmaterial erfindungsgemäß geschont wird; es ist viel mehr gesundes Knochenmaterial übrig, wenn das Freiform-Verfahren gewählt wird.

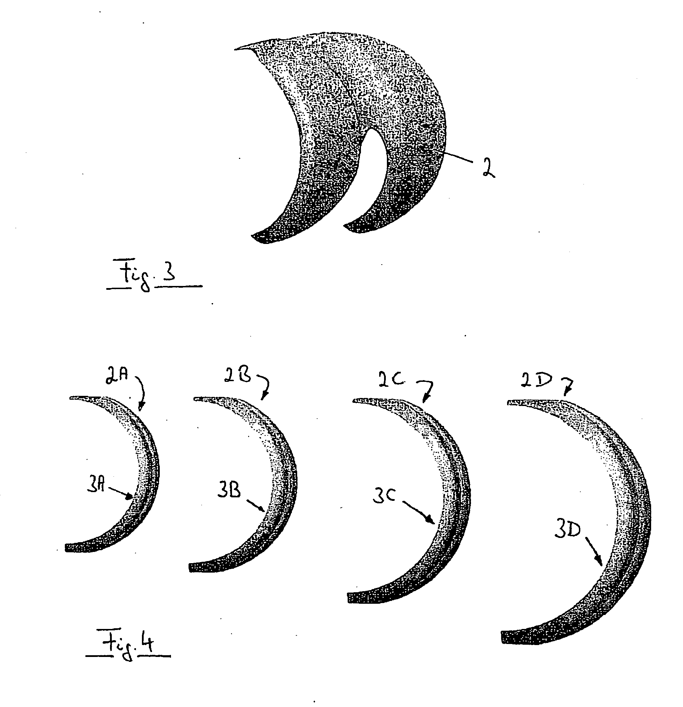

In den Figuren 3 und 4 sind Implantate in verschiedenen Ausführungsformen dargestellt, und zwar in perspektivischer Ansicht. Das in Figur 3 dargestellte Implantat 2 hat eine relativ komplizierte, an die Notwendigkeiten angepasste Außenform und eine Innenform, die zu der erzeugten Freiformfläche passt.

Es wird durch die grundsätzliche Verwendung einer Freiform immer sehr viel mehr Knochenmaterial stehen bleiben als bei Methoden gemäß dem Stand der Technik.

Eine Auswahl von Implantaten 2A bis 2D mit verschiedenen Gesamtgrößen und auch unterschiedlichen Innenkonturen 3A bis 3D ist in Figur 4 gezeigt. Wenn nach der Bilderfassung klar ist, wie die größte funktionelle Außenfläche des Knochens aussehen würde, kann eines der Implantate 2A bis 2D ausgewählt werden, und zwar das jenige, das der Außenkontur nach und der Innenkontur nach schon am besten zur "gesunden Knochenoberfläche" passt.

In bestimmten Fällen ist es auch möglich, das Implantat zusätzlich anzupassen und zwar an die Innen- und/oder Außenfläche.

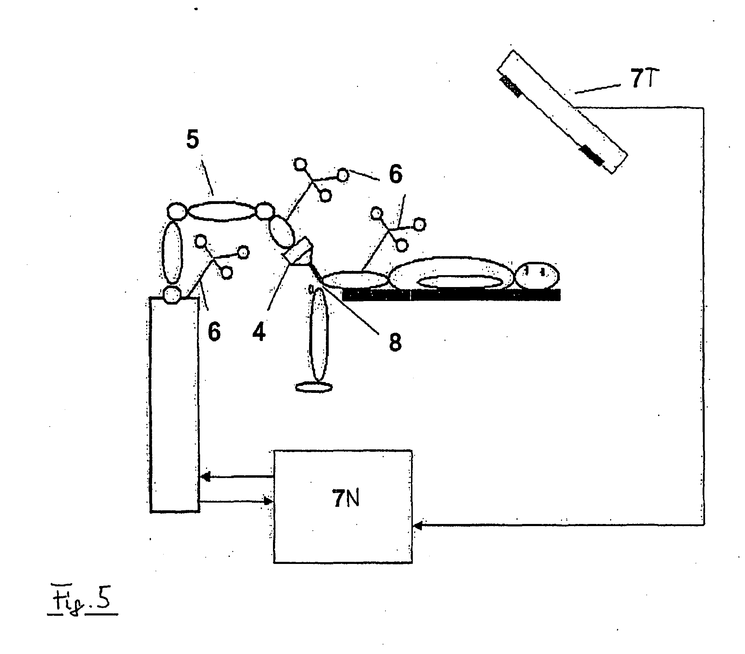

Die Operation mit der erfindungsgemäßen Planungsunterstützung kann mit einem erfindungsgemäßen System durchgeführt werden, das in einer Ausführungsform in der Figur 5 gezeigt ist. Die Figur 5 zeigt ein Laser-Ablationswerkzeug 4, das an dem Arm 5 eines chirurgischen Roboters angebracht ist. Der Roboter trägt sowohl am unteren Basiskörper als auch an dem letzten Arm-Glied Referenzeinrichtungen 6, die durch ein Kamara-Trackingsystem 7T positionell geortet und verfolgt werden können. Das Trackingsystem 7T ist einem Navigationssystem 7N zugeordnet, und das Navigationssystem 7N tauscht Daten mit dem Roboter aus und empfängt Daten von dem Trackingsystem 7T. Der Roboter kann beispielsweise Daten über die Position seiner Gelenke oder des Lasers 4 an das Navigationssystem 7N mitteilen, wobei diese Positionsinformationen aus Gelenksensoren stammen. Andererseits kann das Navigationssystem 7N dem Roboter Positionierungsanweisungen übermitteln, das heißt es kann ihm mitteilen, wie das Werkzeug 4 zu führen ist, und insbesondere auch wohin es nicht geführt werden darf oder wann es ein- oder ausgeschaltet werden muss.

Als sekundäre oder redundante Positionserfassung wird der Roboter noch über die beiden Referenzanordnungen 6 vom Trackingsystem 7T getrackt, die an seiner Basis und an seinem letzten Armglied angeordnet sind.

Ferner ist in der Figur 5 noch zu sehen, dass der Laser 4 am Ende des Oberschenkelknochens eines Patienten arbeitet, wobei die gerade vom Laser 4 bearbeitete Stelle mit dem Bezugszeichen 8 gekennzeichnet ist. Auch der Oberschenkel bzw. Oberschenkelknochen des Patienten wird von Navigations-/-Trackingsystem positionell verfolgt und ist dort registriert, so dass der Arbeitsablauf beim Erzeugen der Freiformfläche exakt nach der erfindungsgemäßen Planung ablaufen kann.

Ein solcher Arbeitsablauf, der unabhängig vom erfindungsgemäßen Planungsverfahren und zeitlich nach diesem durchgeführt wird, kann wie folgt aussehen: Wenn eine Gesamt-Knieersatz-Operation, oder ein Teilersatz (beispielsweise nur eines Gelenkhöckers) vorgesehen ist, besteht der erste Schritt darin, sich durch das Gewebe Zugang zu dem Gelenk zu verschaffen. Als nächstes wird die Oberfläche, die ersetzt werden soll, registriert. Daraufhin wird gemäß der geplanten Abtragungstiefe das nächst-passende Implantat ausgewählt und das disfunktionale Gewebe wird wie geplant abgetragen, beispielsweise durch Laserschneiden, Strahlschneiden oder Hybridlaserschneiden. Weil diese Technologien dazu in der Lage sind, eine freie Form herzustellen, kann die Form des verbleibenden Knochens gut angepasst werden, beispielsweise an die Innenform eines separat für den Patienten hergestellten Implantats.

## Patentansprüche

1. Planungsverfahren zur Anpassung eines Knochenimplantats (2), bei dem
- der Zustand eines zu behandelnden Knochens (1) durch ein bildgebendes Verfahren, insbesondere durch ein Röntgen-, Computertomographie- oder Magnetresonanztomographieverfahren, ermittelt wird;
- durch eine computergraphisch gestützte Segmentierung ein disfunktionaler Teil des Knochens (1) bestimmt wird;
- diejenige Fläche des Knochens (1) als Fläche (3) mit freier Form ermittelt und registriert wird, die gerade unterhalb der Grenzfläche zwischen gesundem Knochengewebe und dem disfunktionalen Teil liegt; und bei dem
- ein Steuerungsdatensatz erstellt wird, welcher der Abtragung des Knochens an der ermittelten Freiformfläche (3) durch eine navigationstechnisch unterstützte Abtragungsvorrichtung (4) zugrunde gelegt werden kann.

2. Planungsverfahren nach Anspruch 1, bei dem der Steuerungsdatensatz für die Führung einer mit freier Hand geführten Abtragungsvorrichtung erstellt wird, die mittels eines medizintechnischen Trackingsystems positionell erfasst und verfolgt wird, wobei auf der Basis des Steuerungsdatensatzes unterstützende Informationen und Anweisungen für die Führung der Abtragungsvorrichtung, die insbesondere über eine Bildausgabe ausgebbar sind, durch ein dem Trackingsystem zugeordnetes medizintechnisches Navigationssystem erstellt werden.

3. Planungsverfahren nach Anspruch 1, bei dem der Steuerungsdatensatz für die Führung einer von Hand führbaren und mechanisch gehalterten Abtragungsvorrichtung erstellt wird, die mittels eines medizintechnischen Trackingsystems positionell erfasst und verfolgt wird, wobei auf der Basis des Steuerungsdatensatzes unterstützende Informationen, Anweisungen sowie insbesondere auch mechanische Führungshilfen für die Führung der Abtragungsvorrichtung durch ein dem Trackingsystem zugeordnetes medizintechnisches Navigationssystem erstellt werden.

4. Planungsverfahren nach Anspruch 1, bei dem der Steuerungsdatensatz für die Führung einer mechanisch gehalterten und geführten Abtragungsvorrichtung erstellt wird, die mittels eines medizintechnischen Trackingsystems positionell erfasst und verfolgt wird, wobei auf der Basis des Steuerungsdatensatzes Steuerungsanweisungen für die Führung der Abtragungsvorrichtung durch ein dem Trackingsystem zugeordnetes medizintechnisches Navigationssystem erstellt werden.

5. Planungsverfahren nach Anspruch 3 oder 4, bei dem der Steuerungsdatensatz für die mechanische Halterung bzw. Führung für einen chirurgischen Roboter erstellt wird, der die Abtragungsvorrichtung trägt.

6. Planungsverfahren nach einem der Ansprüche 1 bis 5, bei dem der Steuerungsdatensatz für eine Abtragungsvorrichtung erstellt wird, die ein Schneidwerkzeug, ein Bohrwerkzeug, ein Fräswerkzeug, ein Laser-Abtragungswerkzeug, ein Strahlschneidwerkzeug oder eine Kombination aus zweien oder mehreren dieser Werkzeuge aufweist.

7. System zur Anpassung eines Knochenimplantats (2) mit Mittlen zur Ermittlung des Zustandes eines zu behandelnden Knochens (1) **durch** ein bildgebendes Verfahren;
- einer Datenverarbeitungseinrichtung mit Mitteln zur Erstellung eines Steuerungsdatensatzes, welcher die Abtragung eines Knochens an einer Freiformfläche (3) steuert,
- Mitteln zur Bestimmung eines disfunktionalen Knochenteils durch computergraphisch gestützte Segmentierung, und mit
- einer navigationstechnisch unterstützten Knochen-Abfragungsvorrichtung (4), welche den Knochen (1) an der Freiformfläche (3) abtragen kann, **dadurch gekennzeichnet, dass** die Datenverarbeitungseinrichtung Mittel zur Ermiltung und Registrierung der Freiformfläche aufweist, die gerade unterhalb der Grenzfläche zwischen gesundem Knochengewebe und dem durch eine computergraphisch gestützte Segmentierung bestimmten disfunktionalen Knochenteil liegt.

8. System nach Anspruch 7, **dadurch gekennzeichnet, dass** es eine Implantat-Bearbeitungsvorrichtung umfasst, die einen Knochen-Anlageabschnitt eines Knochenimplantats (2) als passendes Gegenstück zu der Freiformfläche (3) ausbilden kann.

9. System nach Anspruch 7 oder 8, **dadurch gekennzeichnet, dass** es ein medizintechnisches Trackingsystem umfasst, mit dem die Abtragungsvorrichtung positionell erfasst und verfolgt wird, sowie ein dem Trackingsystem zugeordnetes medizintechnisches Navigationssystem, das auf der Basis des Steuerungsdatensatzes eine Führungsunterstützung für die Abtragungsvorrichtung bereitstellt.

10. System nach einem der Ansprüche 7 bis 9, **dadurch gekennzeichnet, dass** die Abtragungsvorrichtung eine mit freier Hand geführte Abtragungsvorrichtung, eine von Hand führbare und mechanisch gehalterten Abtragungsvorrichtung oder eine mechanisch gehalterte und geführte Abtragungsvorrichtung ist.

11. System nach Anspruch 9 oder 10, **dadurch gekennzeichnet, dass** die Abtragungsvorrichtung von einem chirurgischen Roboter getragen wird, der sie mechanische haltert bzw. führt.

12. System nach einem der Ansprüche 7 bis 11, **dadurch gekennzeichnet, dass** die Abtragungsvorrichtung ein Schneidwerkzeug, ein Bohrwerkzeug, ein Fräswerkzeug, ein Laser-Abtragungswerkzeug, ein Strahlschneidewerkzeug oder eine Kombination aus zweien oder mehreren dieser Werkzeuge aufweist.

## Claims

1. A planning method for modifying a bone implant (2), wherein:
- the condition of a bone (1) to be treated is ascertained by an imaging method, in particular by an x-ray method, a computer tomography method or a magnetic resonance tomography method;
- a dysfunctional part of the bone (1) is determined by computer-graphically assisted segmentation;
- the area of the bone (1) which lies just below the boundary area between healthy bone tissue and the dysfunctional part is ascertained and registered as a free-form area (3); and wherein
- a control data set is produced which can form the basis for ablating the bone on the ascertained free-form area (3) using a navigation-assisted ablating device (4).

2. The planning method according to claim 1, wherein the control data set is produced for guiding an ablating device which is guided by a free hand and is positionally detected and tracked by means of a medical tracking system, wherein on the basis of the control data set, assisting information and instructions for guiding the ablating device, which can in particular be outputted via an image output, are produced by a medical navigation system assigned to the tracking system.

3. The planning method according to claim 1, wherein the control data set is produced for guiding an ablating device which can be guided by hand and is mechanically mounted, and which is positionally detected and tracked by means of a medical tracking system, wherein on the basis of the control data set, assisting information, instructions and in particular also mechanical guiding aids for guiding the ablating device are produced by a medical navigation system assigned to the tracking system.

4. The planning method according to claim 1, wherein the control data set is produced for guiding an ablating device which is mechanically mounted and guided and is positionally detected and tracked by means of a medical tracking system, wherein on the basis of the control data set, control instructions for guiding the ablating device are produced by a medical navigation system assigned to the tracking system.

5. The planning method according to claim 3 or 4, wherein the control data set for mechanically mounting or guiding is produced for a surgical robot which bears the ablating device.

6. The planning method according to any one of claims 1 to 5, wherein the control data set is produced for an ablating device which comprises a cutting tool, a drilling tool, a milling tool, a laser ablating tool, a jet cutting tool or a combination of two or more of these tools.

7. A system for modifying a bone implant (2), comprising:
- means for ascertaining the condition of a bone (1) to be treated, by an imaging method;
- a data processing means, comprising means for producing a control data set which controls the ablation of a bone on a free-form area (3);
- means for determining a dysfunctional part of the bone (1) by computer-graphically assisted segmentation; and comprising
- a navigation-assisted device bone ablating device (4) which can ablate the bone (1) on the free-form area (3), **characterised in that** the data processing means comprises means for ascertaining and registering the free-form area (3) which lies just below the boundary area between healthy bone tissue and the dysfunctional part of the bone (1) determined by computer-graphically assisted segmentation.

8. The system according to claim 7, **characterised in that** it comprises an implant machining device which can form a bone abutting portion of a bone implant (2) as a counter piece which fits the free-form area (3).

9. The system according to claim 7 or 8, **characterised in that** it comprises a medical tracking system using which the ablating device is positionally detected and tracked, and a medical navigation system which is assigned to the tracking system and provides a guiding assistance for the ablating device on the basis of the control data set.

10. The system according to any one of claims 7 to 9, **characterised in that** the ablating device is an ablating device which is guided by a free hand, an ablating device which can be guided by hand and is mechanically mounted, or an ablating device which is mechanically mounted and guided.

11. The system according to claim 9 or 10, **characterised in that** the ablating device is borne by a surgical robot which mechanically mounts or guides it.

12. The system according to any one of claims 7 to 11, **characterised in that** the ablating device comprises a cutting tool, a drilling tool, a milling tool, a laser ablating tool, a jet cutting tool or a combination of two or more of these tools.

## Revendications

1. Procédé de planification pour l'adaptation d'un implant osseux (2), dans lequel
- on détermine l'état d'un os (1) à traiter par un procédé d'imagerie, en particulier par un procédé radiographique, de tomographie assistée par ordinateur ou de tomographie à résonance magnétique ;
- on détermine une partie dysfonctionnelle de l'os (1) par une segmentation assistée graphiquement par ordinateur ;
- on détermine et enregistre la surface de l'os (1), en tant que surface (3) de forme libre, qui se situe précisément au-dessous de la surface limite entre le tissu osseux sain et la partie dysfonctionnelle ; et dans lequel
- on établit un ensemble de données de commande qui peut servir de base à l'enlèvement de l'os sur la surface de forme libre (3) déterminée, au moyen d'un dispositif d'enlèvement (4) assisté par une technique de navigation.

2. Procédé de planification selon la revendication 1 dans lequel on établit l'ensemble de données de commande pour le guidage d'un dispositif d'enlèvement, guidé d'une main libre, dont on détermine la position et que l'on suit au moyen d'un système de poursuite médical, des informations d'assistance et des instructions pour le guidage du dispositif d'enlèvement lesquelles peuvent être éditées en particulier par une sortie image, étant établies par un système de navigation médical associé au système de poursuite.

3. Procédé de planification selon la revendication 1, dans lequel on établit l'ensemble de données de commande pour le guidage d'un dispositif d'enlèvement à guider manuellement et fixé mécaniquement, dont la position est relevée et qui est suivi au moyen d'un système de poursuite médical, des informations d'assistance, des instructions ainsi qu'en particulier aussi des aides mécaniques ou guidages pour le guidage du dispositif d'enlèvement étant établis sur la base de l'ensemble de données de commande par un système de navigation médical associé au système de poursuite.

4. Procédé de planification selon la revendication 1, dans lequel on établit l'ensemble de données de commande pour le guidage d'un dispositif d'enlèvement fixé et guidé mécaniquement, dont la position est relevée et qui est suivi au moyen d'un système de poursuite médical, des instructions de commande pour le guidage du dispositif d'enlèvement étant établies, sur la base de l'ensemble de données de commande, par un système de navigation médical associé au système de poursuite.

5. Procédé de planification selon l'une quelconque des revendications 3 ou 4, dans lequel on établit l'ensemble de données de commande pour la fixation mécanique ou le guidage d'un robot chirurgical, lequel porte le dispositif d'enlèvement.

6. Procédé de planification selon l'une quelconque des revendications 1 à 5, dans lequel on établit l'ensemble de données de commande pour un dispositif d'enlèvement qui comporte un outil de coupe, un outil de perçage, un outil de fraisage, un outil d'enlèvement à laser, un outil de coupe à jet ou une combinaison de deux ou plus de ces outils.

7. Système d'adaptation d'un implant osseux (2) comportant
- des moyens pour déterminer l'état d'un os (1) à traiter par un procédé d'imagerie ;
- un dispositif informatique avec des moyens pour établir un ensemble de données de commande qui commande l'enlèvement d'un os sur une surface de forme libre (3),
- des moyens pour déterminer une partie d'os dysfonctionnelle par une segmentation assistée graphiquement par ordinateur, et comportant
- un dispositif d'enlèvement d'os (4) assisté par une technique de navigation, qui peut enlever l'os (1) sur la surface de forme libre (3),
**caractérisé en ce que** le dispositif informatique (3) comporte des moyens pour déterminer et positionner la surface de forme libre qui se situe précisément au-dessous de la surface limite entre le tissu osseux sain et la partie d'os dysfonctionnelle déterminée par une segmentation assistée graphiquement par ordinateur.

8. Système selon la revendication 7, **caractérisé en ce qu'**il comprend un dispositif d'usinage d'implant qui peut réaliser un segment de contact avec l'os d'un implant osseux (2) en tant que contre-pièce adaptée à la surface de forme libre (3).

9. Système selon l'une quelconque des revendications 7 ou 8, **caractérisé en ce qu'**il comprend un système de poursuite médical qui permet de relever la position et de suivre le dispositif d'enlèvement, ainsi qu'un système de navigation médical associé au système de poursuite qui fournit une assistance au guidage, sur la base de l'ensemble de données de commande, pour le dispositif d'enlèvement.

10. Système selon l'une quelconque des revendications 7 à 9, **caractérisé en ce que** le dispositif d'enlèvement est un dispositif d'enlèvement guidé d'une main libre, un dispositif d'enlèvement à guider manuellement et fixé mécaniquement ou un dispositif d'enlèvement fixé et guidé mécaniquement.

11. Système selon l'une quelconque des revendications 9 ou 10, **caractérisé en ce que** le dispositif d'enlèvement est porté par un robot chirurgical qui le fixe ou guide mécaniquement.

12. Système selon l'une quelconque des revendications 7 à 11, **caractérisé en ce que** le dispositif d'enlèvement comporte un outil de coupe, un outil de perçage, un outil de fraisage, un outil d'enlèvement à laser, un outil de coupe à jet ou une combinaison de deux ou plus de ces outils.
